Europäisches Patentamt

European Patent Office

Office européen des brevets

(11) Publication number: **0 078 859**
**B1**

(12) **EUROPEAN PATENT SPECIFICATION**

(45) Date of publication of patent specification: **26.02.86**

(21) Application number: **81109559.5**

(22) Date of filing: **06.11.81**

(51) Int. Cl.⁴: **C 12 P 9/00, A 61 K 33/26 //**
**(C12P9/00, C12R1:645, 1:85,**
**1:865)**

(54) **An antianaemic agent and a process for producing the same.**

(43) Date of publication of application:
**18.05.83 Bulletin 83/20**

(45) Publication of the grant of the patent:
**26.02.86 Bulletin 86/09**

(84) Designated Contracting States:
**AT BE CH DE FR GB IT LI LU NL SE**

(56) References cited:
**FR-M- 2 130**

**CHIMIA, vol. 16, April 1962, page 142, right-hand column, H.R.SAVERLANDER & CO. AAVER; W. KELLER-SCHIERLEIN: "(Laboratorium für Organische Chemie der ETH, Zurich), Über biologisch wirksame Eisen (III)-trihydroxamat-Komplexe aus Mikroorganismen"**

**ABSTRACTS OF DERWENT,No.19680S, JP-A-7109715**

**CHEMICAL ABSTRACTS, vol. 53, no. 1, 10th January 1959, column 657c-d, Columbus Ohio (USA);**

(73) Proprietor: **Tanaka, Kentaro**
**1-26, Misaki 1-chome Kofu-shi**
**Yamanashi-ken (JP)**

(72) Inventor: **Tanaka, Kentaro**
**1-26, Misaki 1-chome Kofu-shi**
**Yamanashi-ken (JP)**

(74) Representative: **Schübel-Hopf, Ursula et al**
**Strehl, Schübel-Hopf, Schulz Patentanwälte**
**Widenmayerstrasse 17**
**D-8000 München 22 (DE)**

(56) References cited:

**ROTE LISTE, 1980, Editio Cantor. Aulendorf/Wurtt, (DE), Sec. 08, No.08 028B, "Ferronovin"**

Courier Press, Leamington Spa, England.

# 0 078 859

**Description**

This invention relates to an antianaemic agent, more particularly, an organoiron(II) compound-containing antianaemic composition which can be well absorbed into the living body and is chemically stable.

Since iron is a major constituent of hemoglobin, various kinds of iron compounds are used as an antianaemics. Heretofore, a considerable number of researches have been made on the absorption of iron into a living body through the stomach and intestine organs. As a result, it is known that $Fe^{++}$ can be well absorbed in the human organs but $Fe^{+++}$ is not. Accordingly, divalent iron compounds such as ferrous sulfate are most frequently used as a chalybeate. When using divalent iron compounds as a chalybeate, however, special pharmaceutical techniques such as coating should be applied because they are readily oxidized to trivalent iron compounds. It is also known that an iron complex with an organic acid, chelating agent, sugar, amino acid or the like is better absorbed into a living body than inorganic iron compounds. Practically, an organic acid salt of a divalent iron such as ferrous fumarate or ferrous succinate, or a divalent iron chelate has been used for the remedy of a patient suffering from anaemia. Such ferrous compounds, however, still have a disadvantage that they tend to easily be oxidized to the corresponding ferric compounds during the storage or in the living body even after administration thereof to a patient.

On the other hand, wine has long been believed to be effective for the remedy of a patient suffering from anaemia, but any elucidation has not yet been made as to whether the iron values contained in wine are really effective as a chalybeate, or as to in what state the iron values in wine exert an antianaemic effect.

In the article "Studies of Biosynthesis of Iron Containing Substances by Yeast" by Noritaka Shijo and Kentaro Tanaka in "Bulletin of the Research Institute of Fermentation", Yamanashi University, Japan, No. 12, 47—54 (1966) there have been described studies on the fate of soluble iron in fermenting musts of white and red wines into which $^{59}Fe\text{-}FeSO_4$, $^{59}Fe\text{-}FeCl_3$ or $^{59}Fe\text{-}FeCl_3$ together with an amino acid were added. The reference shows that inorganic iron compounds in the musts seem to be incorporated into the yeast during the fermentation and then excreted into the musts as soluble organo iron compounds. In this reference, however, there is no description or suggestion from which the conclusion might be derived that the iron compounds contained in the musts may be useful for preparing an antianaemic composition. After publication of this reference there was still no knowledge about the capability of a composition, derived from iron compound containing musts of being absorbed in the living body of a human being and incorporated into hemoglobin. Further there was no knowledge about the stability of organo iron compounds derived from musts, especially against oxidation, as compared with known divalent iron compounds, such as ferrous chloride, ferrosulfate, ferrofumarate, ferrosuccinate and ferrotartrate.

Starting from the above-described prior art it is an object of the present invention to provide an organoiron(II) compound-containing antianaemic agent which can be efficiently absorbed into a living body and is chemically stable, and which may be prepared by a process which can be carried out easily at low cost.

According to the invention there is provided an anti-anaemic agent comprising an organoiron (II) compound-containing composition, which is produced by a process comprising cultivating a yeast in a saccharide-containing nutrient medium therefor in the presence of an iron compound.

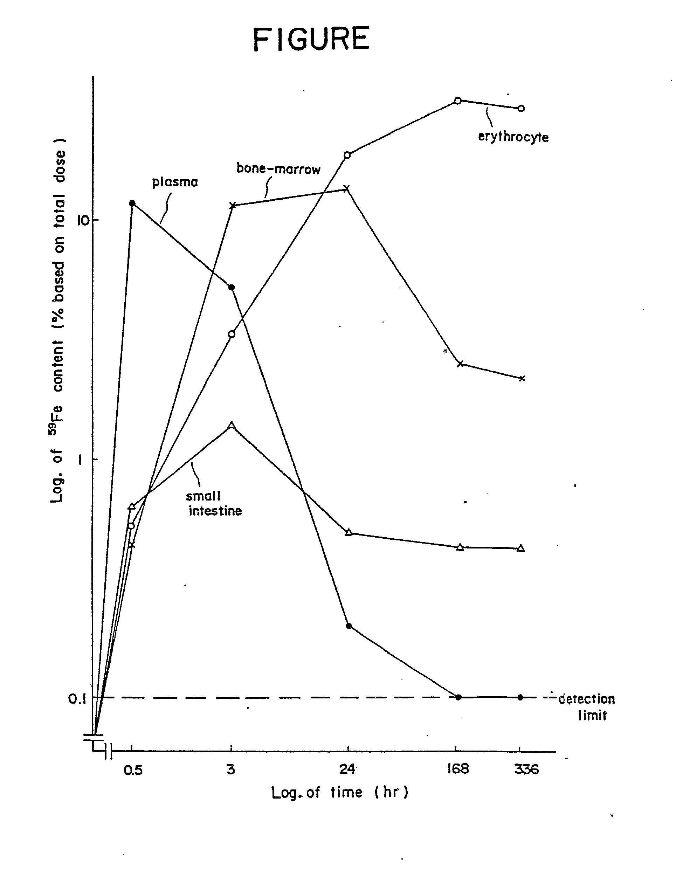

The foregoing and other objects, features and advantages of the present invention will be apparent to those skilled in the art from the following detailed description taken in connection with the accompanying drawing in which:

Figure shows graphs of the relationships between the iron contents in the living body at its plasma, bone-marrow, erythrocyte and small intestine and the lapse of time after administration of the present antianaemic composition.

The kind of a yeast is not critical, but a wide variety of genera ordinarily employed in fermentation may be employed. Preferable genus is *Saccharomyces* or *Metschnikowia*. Examples of species of yeast which may preferably be employed are shown in Table 1.

2

**0 078 859**

TABLE 1

| Species | Strain |
|---|---|
| 1. *Saccharomyces cerevisiae* | OC-2 |
| 2. *Metschnikowia pulcherrima* | K-427 |
| 3. *Saccharomyces cheralieri* | IAM-4861 |
| 4. *Saccharomyces bayanus* | PW-15 |
| 5. *Saccharomyces italicus* | PW-27 |
| 6. *Saccharomyces fermentati* | WF-107 |
| 7. *Saccharomyces rosei* | 28-t |
| 8. *Saccharomyces heterogenicus* | PW-25 |

Note:

The species and strains listed in Table 1 are all known. Of the above-mentioned species, Saccharomyces cerevisiae is most preferred because it can produce a large quantity of a fraction *B* having an excellent absorbability, through the intestine as will be explained later.

Examples of the saccharide-containing nutrient medium to be employed in the present invention include fruit juice, semi-synthetic culture medium and synthetic culture medium. Specific examples of the fruit juice include grape juice (or must), apple juice and the like. Of substances of saccharide, saccharose such as sucrose, glucose, fructose or the like may more preferably be employed.

Preferred examples of semi-synthetic medium and synthetic nutrient medium are given in Tables 2 and 3, respectively.

TABLE 2
(Semi-synthetic)

| | |
|---|---|
| D-glucose | 100.0 g |
| $(NH_4)_2SO_4$ | 5.0 g |
| $KH_2PO_4$ | 1.0 g |
| $MgSO_4 \cdot 7H_2O$ | 0.50 g |
| NaCl | 0.10 g |
| $CaCl_2 \cdot 2H_2O$ | 0.10 g |
| Yeast extract | 1.0 g |
| Distilled water | 1000 ml |
| Initial pH | 6.0 |

3

TABLE 3
(Synthetic)

| | |
|---|---|
| D-glucose | 150 g |
| $(NH_4)_2SO_4$ | 3.5 g |
| $KH_2PO_4$ | 1.0 g |
| $MgSO_4 \cdot 7H_2O$ | 0.50 g |
| NaCl | 0.10 g |
| $CaCl_2 \cdot 2H_2O$ | 0.1 g |
| $H_3BO_3$ | 500 µg |
| $CuSO_4 \cdot 5H_2O$ | 40 µg |
| KI | 100 µg |
| $FeCl_3 \cdot 6H_2O$ | 242 mg |
| $MnSO_4 \cdot H_2O$ | 400 µg |
| $Na_2MoO_4 \cdot 2H_2O$ | 200 µg |
| Biotin | 2 µg |
| $ZnSO_4 \cdot 7H_2O$ | 400 µg |
| Calcium pantothenate | 400 µg |
| Inositol | 2000 µg |
| Thiamine · HCl | 400 µg |
| Distilled water | 1000 ml |
| Initial pH | 7.0 |

The kind of artificial nutrient medium, of course, is not limited to the above-mentioned examples, and any formulations employable in an ordinary fermentation using saccharide-containing nutrient medium may be employed.

As the iron compound, any of those which can be ionized with respect to iron may be employed. As specific examples, there can be mentioned inorganic acid salts of iron such as ferric chloride, ferrous sulfate and ferric sulfate and organic acid salts of iron such as iron citrate and iron tartrate. Iron powder is also useful, but disadvantageously needs a long period of time for its dissolution into a culture medium. From viewpoints of solubility, availability and non-toxicity, the above-mentioned iron compounds preferred. Such an iron compound, when it is of trivalent iron, is converted to produce ferrous ions during the fermentation.

The amount of the iron compound to be added is not critical, but even if the amount of iron added is increased, the amount of iron in the product is not necessarily increased in proportional relationship. Further, the addition of too large an amount of the iron compound adversely suppresses the progress of fermentation. On the other hand, the use of too small an amount of the iron compound cannot exert the intended effect. Usually, the iron compound may be employed in the nutrient medium at a concentration of about 10 to about 70 ppm, more preferably about 30 to about 50 ppm in terms of amount of iron.

In practicing the present invention, whether the fruit juice, semi-synthetic culture medium or synthetic medium is used, the yeast may be employed at a yeast population of about $5 \times 10^5$ to about $3 \times 10^6$/ml of culture medium.

Further fermentation conditions are given as follows. (1) In the case of a culture medium of fruit juice, the cultivation of a yeast can be carried out according to a customary process for the production of wine. For example, the fruit juice, e.g., grape juice or apple juice, as such, or after a saccharide such as glucose, fructose, sucrose or the like is added thereto in such an amount as will exhibit a refraction saccharide degree of about 20 to about 25, is subjected to treatment with a small amount of a germicide (e.g. about 100

4

ppm of potassium metabisulfite) to kill contaminating microorganisms such as wild yeast. Then, the cultivation may usually be conducted at room temperature to 30°C under atmospheric pressure for about 15 days. (2) In the case of a semi-synthetic culture medium or a synthetic culture medium, the cultivation may be conducted at about 18 to about 30°C under stationary condition or anaerobic condition. The amount of a saccharide in the nutrient medium is not critical. From a viewpoint of provision of better conditions of the desired fermentation, the saccharide may generally be incorporated into the nutrient medium in an amount of about 5 to about 25% by weight, more preferably about 10 to about 15% by weight based on the nutrient medium.

The pH of the artificial nutrient medium has initially a value of about 6 to about 7. However, 3 to 4 hours after the initiation of fermentation, the pH value rapidly decreases to 3.6 to 3.8 (this value does not change thereafter) and then the rate of fermentation remarkably increases.

The antianaemic composition produced by the present process can, first, be obtained in the form of a cultured broth. The culture broth may then be separated into a precipitate and a supernatant, for example, by centrifugation or the like. The supernatant is also one form of the present antianaemic composition. In any of them, there is contained a water-soluble organoiron(II) compound which is believed to be an organic complex of divalent iron and is a fermentation product of iron formed by the initially added iron compound's undergoing a chemical reaction during the cultivation or fermentation. Both the cultured broth and the supernatant as such can be administered. For example, when the culture medium is grape juice, they can be enjoyed by people as an organoiron(II)-containing wine. Alternatively, either of the above-mentioned cultured broth and the supernatant may be concentrated at a temperature not exceeding 30°C under a pressure of about 15 to 20 mmHg to obtain a concentrate having a concentration 10 to 20 times that of the original liquor. The concentrates thus obtained can be provided in the form of, e.g., an elixir. The concentrates may be further concentrated to dryness, and the resultant as such or, if desired, together with a reducing additive such as Vitamin C, may be formulated into the adequate form for oral administration, for example, a tablet, capsule, powder, granule or fine granule form. If desired, these dosage forms may be easily prepared according to conventional technique and may comprise commonly employed excipients, binding agents, disintegrators, lubricants and other pharmaceutical agents. As the excipient, binding agent and/or disintegrator, there may be, for example, mentioned microcrystalline cellulose, wheat starch, sugar, lactose, gum arabic, tragacanth gum, carboxymethylcellulose and so on. As the lubricant, there may be given, e.g., magnesium stearate and talc. Tablets may be also coated according to conventional coating procedures and any commonly employable coating materials such as, for example, shellac, ethylcellulose, hydroxymethylcellulose, polyvinyl pyrrolidone, titanium dioxide and the like may be favorably applied for such purposes. The dosage may vary depending upon ages, severities and body weights of patients, but the present antianaemic composition may be usually administered in a daily dose of from about 5 mg to about 100 mg in terms of amount of iron for adults, if necessary, in divided dosage forms.

Thus, in another aspect of the present invention, there is provided an organoiron(II) compound-containing antianaemic composition produced by a process comprising cultivating a yeast in a saccharide-containing nutrient medium therefor in the presence of an iron compound.

As stated before, from the chemical analysis, it is believed that the active ingredient of the present antianaemic composition is a divalent iron-containing organic complex composed only of elements Fe, C, H and O whose molecular weight is in the range of about $10^2$ to about $5\times10^3$. As is apparent from Experiment which will be given later, it is clearly recognized that the organoiron(II) in the present antianaemic composition is much more effective in capability of being absorbed in the living body of a human being and animals and incorporation into hemoglobin than the conventional chalybeates. Furthermore, it is clearly recognized that the organoiron(II) in the present antianaemic composition is remarkably stable as compared with divalent iron compounds such as ferrous chloride, ferrous sulfate, ferrous fumarate, ferrous succinate and ferrous tartrate.

When the supernatant as mentioned before is subjected to chromatographic fractionation, for example, by means of Amberlite (Trade Mark) XAD-2 (available from Rohm and Haas Co., U.S.A.), it is separated into two fractions, namely, Fraction $A$ and Fraction $B$. In this connection, Fraction $B$ has a relatively low molecular weight but a high absorbability into the living body as compared with Fraction $A$.

In still another aspect of the present invention, there is provided a method of increasing an iron content in blood which comprises administering to a patient an effective amount of an organoiron(II) compound-containing antianaemic composition produced by a process comprising cultivating a yeast in a saccharide-containing nutrient medium therefor in the presence of an iron compound.

The present invention will be illustrated in more detail with reference to the following Examples, which should not be construed to be limiting the scope of the present invention.

Example 1

Sugar was added to grape juice from *Koshu* grape (produced in the vicinity of Kofu-city, Japan) in such an amount as would give a refraction saccharide degree of 24. To the mixture was added potassium metabisulfite in an amount of 100 ppm in terms of amount of sulfurous acid. Then, $FeCl_3$ was added in an amount of 50 ppm in terms of amount of iron, and a yeast mash of *Saccharomyces cerevisiae* OC-2 was added in an amount of 3% by volume based on the grape juice. The mixture was subjected to fermentation at 20°C for 15 days. The resulting fermentation product was subjected to filtration to obtain wine. The iron content of the wine was 31 ppm.

Experiments

Animal tests were conducted to show the effectiveness of the iron values in the wine prepared according to the present invention.

1) Preparation of test sample:

(a) Wine according to the present invention:

Sugar was added to grape juice from *Koshu* grape in such an amount as would give a refraction saccharide degree of 24. To the mixture was added potassium metabisulfite in an amount of 100 ppm in terms of amount of sulfurous acid. On the other hand, $^{59}FeCl_3$ was added to a solution of ferric chloride in 0.1N hydrochloric acid in such an amount as would give a specific radioactivity of 6.25 µCi/50 µgFe, and the pH of the mixture was adjusted to pH 3 by adding an aqueous 0.1N sodium hydroxide solution. This was added to the above-prepared grape juice medium in an amount of 50 ppm in terms of amount of iron. Then, a yeast mash of *Saccharomyces cerevisiae* OC-2 was added in an amount of 3% by volume based on the iron-containing grape juice medium. Fermentation was conducted at about 20°C. After completion of the intended fermentation, the mixture was subjected to centrifugation at 10,000 rpm for 10 minutes to obtain wine containing $^{59}Fe$. By expelling alcohol under reduced pressure at 30°C, test samples respectively containing 10, 40, 80 and 250 µgFe/ml were obtained.

(b) Ferrous sulfate solution (Comparative)

Put into the $^{59}FeCl_3$ solution with a specific radioactivity of 12.5 µCi/µgFe was ascorbic acid in an amount of 0.05 µ mol per µg of iron to give divalent iron. By mixing this with an aqueous ferrous sulfate solution, comparative test samples having 5 µCi/10, 40, 80 and 250 µgFe/ml and a pH value of 3 were obtained.

(c) Ferric chloride solution (Comparative)

Put into the $^{59}FeCl_3$ solution was an aqueous ferric chloride solution, and the pH of the mixture was adjusted to pH 3 by adding an aqueous sodium hydroxide solution. Comparative test samples having 5 µCi/10, 40, 80 and 250 µgFe/ml were obtained.

(d) Concentrated wine-ferrous sulfate mixture (Comparative)

Wine (in which the iron originating in grape juice is present in an amount of about 1 ppm) prepared in the same procedures as described in Example except that $FeCl_3$ was not added, was concentrated to 1/4 in volume. By mixing this and the ferrous sulfate solution (prepared according to the above-mentioned procedures and having 10 µCi/80 µgFe/ml) at a ratio of 1:1 by volume, a comparative test sample having 5 µCi/40 µgFe/ml was obtained.

2) Administration:

Five-week old male Sprague-Dawly rats (available from Nippon Kurea K.K., Japan) were subjected to preparatory breeding for one week. They were fed with a feed "CA-1" (containing iron in an amount of 31.5 mg/100 g, and manufactured and sold by Nippon Kurea K.K., Japan), and city water was given.

During the preparatory breeding, weight increase was checked. Those rats having a body weight of about 160 g at the age of six weeks were subjected to the experiments. Through the period of experiments, the rats were bred in a metabolic cage. No feeding was done for 18 hours before administering a test sample, but distilled water was continued to be given. Each test sample prepared according to the above-mentioned procedures was administered to a group of five rats, directly into their stomach by means of stomach probe, in an amount of 1 ml/160 g body weight. Six hours after administering the test sample, feeding of feed "CA-1" was resumed.

3) Determining radioactivity of in-vivo sample:

After administering each test sample, feces and urine were taken at intervals of hours to determine radioactivities thereof. 50 µl of blood was taken by means of a heparin-treated capillary from the tail vein at intervals of hours, and subjected to centrifugation at 3,000 rpm for 5 minutes to separate blood corpuscles from plasma. Radioactivities of the so obtained blood corpuscles and plasma were determined. Absorption of iron into the body and incorporation of the body-absorbed iron into hemoglobin are shown in Table 4.

TABLE 4

(Comparison between organoiron(II)-containing wine, inorganic ferrous compound and inorganic ferric compound, with respect to absorbability into the living body and incorporation of iron into hemoglobin)

| Iron concentration in sample (µg/ml) | Dose (Fe µg/ kg body weight) | Kind of sample | A | B | B/A |
|---|---|---|---|---|---|
| | | | Absorbability (%) | Incorporation into hemoglobin (%) | Utility of absorbed iron (%) |
| 10 | 62.5 | (a) | 69.4±1.4 | 48.0±1.8 | 69.2±1.5 |
| | | (b) | 67.4±3.6 | 47.5±1.8 | 70.5±2.5 |
| | | (c) | 50.1±4.2 | 32.0±6.2 | 63.9±2.6 |
| 40 | 250 | (a) | 53.5±1.7 | 28.5±2.3 | 53.3±2.5 |
| | | (b) | 37.6±4.0 | 20.0±1.9 | 53.2±4.0 |
| | | (c) | 29.9±6.9 | 15.7±4.2 | 52.5±4.5 |
| | | (d) | 31.0±3.5 | 17.9±3.2 | 57.7±3.5 |
| 80 | 500 | (a) | 35.5±4.6 | 17.1±2.3 | 48.2±4.5 |
| | | (b) | 20.0±4.0 | 10.5±1.9 | 52.5±10.5 |
| | | (c) | 16.5±3.1 | 7.2±2.0 | 43.6±3.1 |
| 250 | 1500 | (a) | 23.5±3.5 | 7.2±0.9 | 30.6±3.0 |
| | | (b) | 9.7±4.5 | 3.8±2.0 | 39.2±8.0 |
| | | (c) | — | — | — |

Note

A (absorbability)=$100\times(1$—amount of discharged $^{59}$Fe/amount of total dose of $^{59}$Fe); and

B (Incorporation into hemoglobin): $^{59}$Fe content in the whole blood at the time of 168 hours after administration of sample.

Each figure represents a mean value ± standard error.

As is apparent from Table 4, when the iron concentration in administered sample of the present composition is as low as about 1 mg/liter or less, there is not a remarkable difference in absorbability into a living body between the organic iron complex according to the present invention and the inorganic iron salts. This is assumed to be attributed to the fact that such an iron concentration as low as about 1 mg/liter or less is within such a range that the reducing capacity of the living body itself is sufficient therefor. But, when the iron concentration is relatively high, the antianaemic composition according to the present invention exhibits a remarkably improved absorbability into the living body as compared with $FeSO_4$ and $FeCl_3$. On the other hand, no significant difference in absorbability into the living body is seen between the mixture of concentrated ordinary wine and ferrous sulfate [i.e. (d) in Table 4] and ferrous sulfate per se [i.e. (c) in Table 1].

Example 2

Substantially the same procedures as in Example 1 were repeated except that a semi-synthetic culture medium of Table 2 was employed as the nutrient medium. The mixture was subjected to fermentation at 30°C for 15 days. The cultured broth was subjected to filtration to obtain a supernatant. The iron content of the supernatant was 35 ppm. The organoiron(II) compound-containing supernatant was very stable and excellent in antianaemic activity.

Example 3

Substantially the same procedures as in Example 1 were repeated except that a synthetic culture medium of Table 3 was employed as the nutrient medium. The mixture was subjected to fermentation at 30°C for 15 days. The cultured broth was subject to filtration to obtain a supernatant. The iron content of the supernatant was 33 ppm. The organoiron(II) compound-containing supernatant was very stable and excellent in antianaemic activity.

Turning now to Figure, there are shown the relationships between the iron contents in the living body at its plasma, bone-marrow, erythrocyte and small intestine and the lapse of time after administration of the sample prepared from the supernatant obtained in Example 2 and containing 250 μm Fe/ml in a total dose of 250 μg Fe/kg·body weight. The tests were carried out in substantially the same manner as in the above-mentioned Experiments, items 2) and 3) to determine the change in iron content in the living body at its plasma, bone-marrow, erythrocyte and small intestine with the lapse of time after the administration. As is clearly seen from Figure, first, the iron content in the plasma rapidly increases but, in turn, rapidly decreases to reach nearly zero about 168 hours after the administration. Instead, second, the iron content in the bone-marrow increases and begins to decrease about 20 hours after the administration. Third, the iron content in the erythrocyte gradually increases to reach a plateau region (in which the iron content is larger than those observed with respect to the plasma and the bone-marrow) and the iron content in the erythrocyte which has once reached the plateau region does not change for about 50 days. The graphs suggest that the iron values absorbed in the plasma are transferred to the erythrocyte through the bone-marrow. The graphs also show that the organoiron(II) compound of the present antianaemic composition is very stably absorbed into the living body and that the incorporation of iron into hemoglobin is very large. Therefore, the times of dosage can advantageously be reduced.

**Claims for the Contracting States: BE, CH, DE, FR, GB, IT, LI, LU, NL, SE**

1. An antianaemic agent comprising an organoiron(II) compound-containing composition, which is produced by a process comprising cultivating a yeast in a saccharide-containing nutrient medium therefor in the presence of an iron compound.

2. An antianaemic agent according to claim 1, wherein the yeast belongs to the genus *Saccharomyces* or *Metschnikowia*.

3. An antianaemic agent according to claim 2, wherein the yeast is *Saccharomyces cerevisiae, Metschnikowia pulcherrima, Saccharomyces chevalieri, Saccharomyces bayanus, Saccharomyces italicus, Saccharomyces fermentati, Saccharomyces rosei* or *Saccharomyces heterogenicus*.

4. An antianaemic agent according to claim 1, wherein the saccharide-containing nutrient medium is a fruit juice, a semi-synthetic culture medium or a synthetic culture medium.

5. An antianaemic agent according to claim 4, wherein the fruit juice is grape juice or apple juice.

6. An antianaemic agent according to claim 1, wherein the cultivation is conducted in the nutrient medium having a yeast population of $5 \times 10^5$ to $3 \times 10^6$/ml of the medium.

7. An antianaemic agent according to claim 1, wherein the iron compound is ferric chloride, ferrous sulfate, ferric sulfate, iron citrate or iron tartrate.

8. An antianaemic agent according to any of claims 1 to 7, wherein the iron compound is incorporated into the nutrient medium in an amount of 10 to 70 ppm in terms of amount of iron.

9. An antianaemic agent according to claim 4, wherein the nutrient medium is a semi-synthetic culture medium or a synthetic culture medium and the saccharide content of the nutrient medium is 5 to 25% by weight.

10. An antianaemic agent according to any of claims 1 to 9, wherein the cultivation is conducted at a temperature of 18 to 30°C.

11. An antianaemic agent according to any of claims 1 to 10, which is produced by a process further comprising concentrating a resulting cultured broth to 1/10 to 1/20 in volume or to dryness.

12. An antianaemic agent according to claims 1 to 10, which is produced by a process further comprising subjecting a resulting cultured broth to separation to isolate a supernatant.

13. An antianaemic agent according to claim 12, which is produced by a process further comprising concentrating the isolated supernatant to 1/10 to 1/20 in volume or to dryness.

## Claims for the Contracting State: AT

1. A process for producing an antianaemic agent comprising an organoiron(II) compound-containing composition which comprises cultivating a yeast in a saccharide-containing nutrient medium therefor in the presence of an iron compound.

2. A process according to claim 1, wherein the yeast belongs to the genus *Saccharomyces* or *Metschnikowia*.

3. A process according to claim 2, wherein the yeast is *Saccharomyces cerevisiae, Metschnikowia pulcherrima, Saccharomyces chevalieri, Saccharomyces bayanus, Saccharomyces italicus, Saccharomyces fermentati, Saccharomyces rosei* or *Saccharomyces heterogenicus*.

4. A process according to claim 1, wherein the saccharide-containing nutrient medium is a fruit juice, a semi-synthetic culture medium or a synthetic culture medium.

5. A process according to claim 4, wherein the fruit juice is grape juice or apple juice.

6. A process according to claim 1, wherein the cultivation is conducted in the nutrient medium having a yeast population of $5 \times 10^5$ to $3 \times 10^6$/ml of the medium.

7. A process according to claim 1, wherein the iron compound is ferric chloride, ferrous sulfate, ferric sulfate, iron citrate or iron tartrate.

8. A process according to any of claims 1 to 7, wherein the iron compound is incorporated into the nutrient medium in an amount of 10 to 70 ppm in terms of amount of iron.

9. A process according to claim 4, wherein the nutrient medium is a semi-synthetic culture medium or a synthetic culture medium and the saccharide content of the nutrient medium is 5 to 25% by weight.

10. A process according to any of claims 1 to 9, wherein the cultivation is conducted at a temperature of 18 to 30°C.

11. A process according to any of claims 1 to 10, which further comprises concentrating a resulting cultured broth to 1/10 to 1/20 in volume or to dryness.

12. A process according to claims 1 to 10, which further comprises subjecting a resulting cultured broth to separation to isolate a supernatant.

13. A process according to claim 12, which further comprises concentrating the isolated supernatant to 1/10 to 1/20 in volume or to dryness.

## Patentansprüche für die Vertragsstaaten BE, CH, DE, FR, GB, IT, LI, LU, NL, SE

1. Antianaemisches Mittel, umfassend eine eine Organoeisen-(II)-Verbindung enthaltende Zusammensetzung, die mit Hilfe eines Verfahrens hergestellt wird, bei dem eine Hefe in einem Saccharid-haltigen Nährmedium für diese in Gegenwart einer Eisenverbindung gezüchtet wird.

2. Antianaemisches Mittel nach Anspruch 1, wobei die Hefe dem Genus Saccharomyces oder Metschnikowia angehört.

3. Antianaemisches Mittel nach Anspruch 2, wobei die Hefe Saccharomyces cerevisiae, Metschnikowia pulcherrima, Saccharomyces chevalieri, Saccharomyces bayanus, Saccharomyces italicus, Saccharomyces fermentati, Saccharomyces rosei oder Saccharomyces heterogenicus ist.

4. Antianaemisches Mittel nach Anspruch 1, wobei das Saccharid-haltige Nährmedium Fruchtsaft, ein halbsynthetisches Kulturmedium oder ein synthetisches Kulturmedium ist.

5. Antianaemisches Mittel nach Anspruch 4, wobei der Fruchtsaft Traubensaft oder Apfelsaft ist.

6. Antianaemisches Mittel nach Anspruch 1, wobei die Züchtung in dem Nährmedium derart durchgeführt iwrd, daß eine Hefepopulation von $5 \times 10^5$ bis $3 \times 10^6$/ml des Mediums vorliegt.

7. Antianaemisches Mittel nach Anspruch 1, wobei die Eisenverbindung Ferrichlorid, Ferrosulfat, Ferrisulfat, Eisencitrat oder Eisentartrat ist.

8. Antianaemisches Mittel nach einem der Ansprüche 1 bis 7, wobei die Eisenverbindung dem Nährmedium in einer Menge von 10 bis 70 ppm, bezogen auf Eisen, einverleibt ist.

9. Antianaemisches Mittel nach Anspruch 4, wobei das Nährmedium ein halbsynthetisches Kulturmedium oder ein synthetisches Kulturmedium ist und der Saccharidgehalt des Nährmediums 5 bis 25 Gew.-% beträgt.

10. Antianaemisches Mittel nach einem der Ansprüche 1 bis 9, wobei die Züchtung bei einer Temperatur von 18 bis 30°C durchgeführt wird.

11. Antianaemisches Mittel nach einem der Ansprüche 1 bis 10, welches mit Hilfe eines Verfahrens erhalten wird, gemäß dem zusätzlich die gebildete Kulturbrühe auf 1/10 bis 1/20 ihres Volumens oder zur Trockene konzentriert wird.

12. Antianaemisches Mittel nach Ansprüchen 1 bis 10, erhalten mit Hilfe eines Verfahrens, gemäß dem

# 0 078 859

die gebildete Kulturbrühe einem Trennvorgang zur Isolierung einer überstehenden Schicht unterworfen wird.

13. Antianaemisches Mittel nach Anspruch 12, erhalten mit Hilfe eines Verfahrens, bei dem zusätzlich die isolierte überstehende Schicht auf 1/10 bis 1/20 ihres Volumens oder zur Trockene konzentriert wird.

## Patentansprüche für den Vertragsstaat AT

1. Verfahren zur Herstellung eines antianaemischen Mittels, das eine eine Organoeisen-(II)-Verbindung enthaltende Zusammensetzung umfaßt, dadurch gekennzeichnet, daß eine Hefe in einem Saccharid-haltigen Nährmedium für diese in Gegenwart einer Eisenverbindung gezüchtet wird.

2. Verfahren nach Anspruch 1, bei dem die Hefe dem Genus Saccharomyces oder Metschnikowia angehört.

3. Verfahren nach Anspruch 2, bei dem die Hefe Saccharomyces cerevisiae, Metschnikowia pulcherrima, Saccharomyces chevalieri, Saccharomyces bayanus, Saccharomyces italicus, Saccharomyces fermentati, Saccharomyces rosei oder Saccharomyces heterogenicus ist.

4. Verfahren nach Anspruch 1, bei dem das Saccharid-haltige Nährmedium ein Fruchtsaft, ein halbsynthetisches Kulturmedium oder ein synthetisches Kulturmedium ist.

5. Verfahren nach Anspruch 4, bei dem der Fruchtsaft Traubensaft oder Apfelsaft ist.

6. Verfahren nach Anspruch 1, bei dem die Züchtung in einem Nährmedium durchgeführt wird, dessen Hefepopulation $5 \times 10^5$ bis $3 \times 10^6$/ml des Mediums beträgt.

7. Verfahren nach Anspruch 1, bei dem die Eisenverbindung Ferrichlorid, Ferrosulfat, Ferrisulfat, Eisencitrat oder Eisentartrat ist.

8. Verfahren nach einem der Ansprüche 1 bis 7, bei dem die Eisenverbindung dem Nährmedium in einer Menge von 10 bis 70 ppm, bezogen auf Eisen, einverleibt wird.

9. Verfahren nach Anspruch 4, bei dem das Nährmedium ein halbsynthetisches Kulturmedium oder ein synthetisches Kulturmedium ist und der Saccharidgehalt des Nährmediums 5 bis 25 Gew.-% beträgt.

10. Verfahren nach einem der Ansprüche 1 bis 9, bei dem die Züchtung bei einer Temperatur von 18 bis 30°C durchgeführt wird.

11. Verfahren nach einem der Ansprüche 1 bis 10, bei dem zusätzlich die erhaltene Kulturbrühe auf 1/10 bis 1/20 ihres Volumens oder zur Trockene konzentriert wird.

12. Verfahren nach Ansprüchen 1 bis 10, bei dem außerdem die gebildete Kulturbrühe der Trennung unterworfen wird, um eine überstehende Schicht zu isolieren.

13. Verfahren nach Anspruch 12, bei dem außerdem die isolierte überstehende Schicht auf 1/10 bis 1/20 ihres Volumens oder zur Trockene konzentriert wird.

## Revendications pour les Etats Contractants: BE, CH, DE, FR, GB, IT, LI, LU, NL, SE

1. Agent antianémique comprenant une composition contenant un composé organofer (II), qui est obtenu par un procédé comprenant la fabrication d'une culture d'une levure dans un milieu nutritif contenant un saccharide et en présence d'un composé du fer.

2. Agent antianémique selon la revendication 1, où la levure appartient au genre saccharomyces ou metschnikowia.

3. Agent antianémique selon la revendication 2, où la levure est Saccharomyces cerevisiae, Metschnikowia pulcherrima, Saccharomyces chevalieri, Saccharomyces bayanus, Saccharomyces italicus, Saccharomyces fermentati, Saccharomyces rosei ou Saccharomyces heterogenicus.

4. Agent antianémique selon la revendication 1, où le milieu nutritif contenant un saccharide est un jus de fruit, un milieu de culture semi-synthétique ou un milieu de culture synthétique.

5. Agent antianémique selon la revendication 4, où le jus de fruit est un jus de raisin ou un jus de pomme.

6. Agent antianémique selon la revendication 1, où la culture est effectuée dans le milieu nutritif ayant une population de levure de $5.10^5$ à $3.10^6$/ml de milieu.

7. Agent antianémique selon la revendication 1, où le composé de fer et du chlorure ferrique, sulfate ferreux, sulfate ferrique, citrate de fer ou tartrate de fer.

8. Agent antianémique selon l'une quelconque des revendications 1 à 7, où le composé de fer est introduit dans le milieu nutritif en une quantité de 10 à 70 ppm en termes de quantité de fer.

9. Agent antianémique selon la revendication 4, où le milieu nutritif est un milieu de culture semi-synthétique ou un milieu de culture synthétique et la teneur en saccharide du milieu nutritif est de 5 à 25% en poids.

10. Agent antianémique selon l'une des revendications 1 à 9, où la culture est effectuée à une température de 18 à 30°C.

11. Agent antianémique selon l'une des revendications 1 à 10, qui est produit par un procédé comprenant de plus la concentration du bouillon de culture résultant jusqu'à 1/10 à 1/20 en volume ou jusqu'à siccité.

12. Agent antianémique selon l'une des revendications 1 à 10, qui est produit par un procédé

10

comprenant de plus la soumission d'un bouillon de culture résultant à une séparation pour isoler un surnageant.

13. Agent antianémique selon la revendication 12, qui est produit par un procédé comprenant de plus la concentration du surnageant isolé jusqu'à 1/10 à 1/20 en volume ou jusqu'à siccité.

**Revendications pour l'Etat Contractant: AT**

1. Procédé pour l'obtention d'un agent antianémique comprenant une composition contenant un composé organofer (II) qui comprend la fabrication d'une culture de levure dans un milieu nutritif contenant du saccharide et en présence d'un composé de fer.

2. Procédé selon la revendication 1, où la levure appartient au genre Saccharomyces ou Metschnikowia.

3. Procédé selon la revendication 2, où la levure est Saccharomyces cerevisiae, Metschnikowia pulcherrima, Saccharomyces chevalieri, Saccharomyces bayanus, Saccharomyces italicus, Saccharomyces fermentati, Saccharomyces rosei ou Saccharomyces heterogenicus.

4. Procédé selon la revendication 1, où le milieu nutritif contenant du saccharide est un jus de fruit, un milieu de culture semi-synthétique ou un milieu de culture synthétique.

5. Procédé selon la revendication 4, où le jus de fruit est un jus de raisin ou un jus de pomme.

6. Procédé selon la revendication 1, où la culture est effectuée dans un milieu nutritif ayant une population de levure de $5.10^5$ à $3.10^6$/ml de milieu.

7. Procédé selon la revendication 1, où le composé de fer est chlorure ferrique, sulfate ferreux, sulfate ferrique, citrate de fer ou tartrate de fer.

8. Procédé selon l'une des revendications 1 à 7, où le composé de fer est introduit dans le milieu nutritif en une quantité de 10 à 70 ppm en termes de quantité de fer.

9. Procédé selon la revendication 4, où le milieu nutritif est un milieu de culture semi-synthétique ou un milieu de culture synthétique et où la teneur en saccharide du milieu nutritif est de 5 à 25% en poids.

10. Procédé selon l'une des revendications 1 à 9, où la culture est effectuée à une température de 18 à 30°C.

11. Procédé selon l'une des revendications 1 à 10, qui comprend de plus la concentration d'un bouillon de culture résultant jusqu'à 1/10 à 1/20 en volume ou jusqu'à siccité.

12. Procédé selon l'une des revendications 1 à 10, qui comprend de plus la soumission du bouillon de culture résultant à séparation pour isoler un surnageant.

13. Procédé selon la revendication 12, qui comprend de plus la concentration du supernageant isolé jusqu'à 1/10 à 1/20 en volume ou jusqu'à siccité.

# FIGURE